# FASCICULE DE BREVET EUROPEEN

(11) **EP 3 003 146 B1**
(45) Date de publication et mention de la délivrance du brevet: **17.07.2019**
(21) Numéro de dépôt: 14726654.8
(22) Date de dépôt: 28.05.2014
(51) Int. Cl.: A61B 5/107, A61B 5/00

(54) **DISPOSITIF DE MESURE DE LA CIRCONFERENCE D'UN OBJET, EN PARTICULIER D'UN MEMBRE CORPOREL**
VORRICHTUNG ZUR MESSUNG DES UMFANGS EINES OBJEKTS, INSBESONDERE EINER GLIEDMASSE
DEVICE FOR MEASURING THE CIRCUMFERENCE OF AN OBJECT, IN PARTICULAR A LIMB

(30) Priorité: 30.05.2013 BE 201300380
(43) Date de publication de la demande: 13.04.2016
(73) Titulaire: Just A New Health, 1320 Beauvechain (BE)
(72) Inventeur: HARFOUCHE, Joseph, B-1190 Forest (BE)
(74) Mandataire: ABYOO
(86) Numéro de dépôt international: PCT/EP2014/061165
(87) Numéro de publication internationale: WO 2014/191513

(56) Documents cités:
- WO-A1-93/14728
- GB-A- 2 452 256
- US-A- 4 754 528

## Description

La présente invention se rapporte à un dispositif de mesure itérative de la circonférence d'un objet, en particulier d'un membre corporel, comprenant :
- un premier élément de mesure gradué longitudinal présentant une première direction longitudinale, agencé pour être apposé le long dudit objet, en particulier le long dudit membre corporel et définissant une coulisse,
- une sangle distale et une sangle proximale inscrite chacune dans un plan sensiblement perpendiculaire à ladite direction longitudinale, et
- un coulisseau coopérant avec ladite coulisse, relié à un deuxième élément de mesure gradué longitudinal, ledit coulisseau présentant un orifice de passage de coulisse et étant positionné entre ladite sangle distale et ladite sangle proximale.

Une mesure de la circonférence de membres corporels tels qu'un bras ou une jambe est notamment recommandée dans le cadre du bilan et du suivi du traitement de kinésithérapie, par exemple pour le traitement des lymphoedèmes, c'est-à-dire de gonflements d'une partie du corps suite à une accumulation de liquide lymphatique dans les tissus interstitiels. De tels gonflements apparaissent lorsque les lymphangions qui constituent les vaisseaux lymphatiques sont dégradés ou non-fonctionnels (lymphoedème primaire) ou lorsque les vaisseaux lymphatiques eux-mêmes sont endommagés ou obstrués et lorsque des nodules lymphatiques ont été enlevés (lymphoedème secondaire).

Plus particulièrement, les lymphoedèmes secondaires sont une conséquence d'un dommage ou d'un traumatisme par exemple causé par un accident, une chirurgie, une infection grave ou encore une radiothérapie ou d'autres causes.

Ces gonflements concernent essentiellement les membres supérieurs et inférieurs tels que par exemple les bras, les pieds, les jambes, les cuisses et les mains mais peuvent aussi se produire au niveau d'autres parties du corps comme le cou, l'abdomen, le dos ou encore les seins. A noter que le lymphoedème secondaire des membres supérieurs est principalement causé par le traitement chirurgical du creux axillaire du cancer du sein consistant en une ablation des ganglions de l'aisselle.

Afin de déterminer dans quelle mesure un lymphoedème (primaire ou secondaire) doit être traité, il convient d'en suivre l'évolution. A titre d'exemple, si on considère le lymphoedème secondaire des membres supérieurs, le guide de pratique clinique pour la prise en charge et le traitement du cancer du sein (Guides de pratique clinique pour la prise en charge et le traitement du cancer du sein, Canadian Médical Association Journal) préconise de mesurer la circonférence branchiale à quatre points : aux articulations métacarpiennes-phalangiennes, aux poignets, à 10 cm en aval et à 15 cm en amont des épicondyles latéraux (coude). Il est considéré qu'une différence de plus de 2 cm de la circonférence, entre deux mesures à l'un de ces quatre points de mesure, justifie le traitement du lymphoedème. Une différence de plus de 2 cm de la circonférence entre un membre corporel (par exemple le bras droit) présentant un lymphoedème et un membre corporel correspondant (par exemple le bras gauche) ne présentant pas de lymphoedème indique également qu'il convient de procéder à un traitement de ce gonflement.

Il est donc nécessaire de disposer d'un dispositif ou d'un instrument de mesure qui permette de mesurer la circonférence de membres corporels ponctuellement et à un même endroit afin d'être en mesure de décider si un traitement du lymphoedème est d'application ou non. Il convient particulièrement de disposer d'un outil de mesure qui permette une mesure précise et fiable puisque la marge d'erreur doit être faible et seulement de l'ordre de quelques millimètres, de préférence de l'ordre de moins de 5 millimètres et plus préférentiellement de l'ordre de moins de 2 millimètres.

Une mesure de la circonférence de membres corporels est également indiquée pour constater une diminution du volume des muscles squelettiques et en suivre l'évolution. Une telle diminution du volume musculaire (ou perte de la masse musculaire) peut par exemple être due à une amyotrophie (atrophie et/ou disparition de la fibre musculaire striée), à une sarcopénie (syndrome gériatrique) ou encore à des myopathies (maladie neuro-musculaires). Ces pathologies nécessitent un suivi et notamment un traitement de kinésithérapie au cours duquel une mesure précise de la circonférence du membre corporel comprenant le muscle considéré est indispensable. Ici aussi, l'évolution de la circonférence du membre corporel peut être suivie par comparaison de deux mesures effectuées à un même endroit après une période de temps prédéterminée ou en comparant les circonférences d'un membre corporel « sain » et d'un membre corporel correspondant présentant une diminution du volume musculaire (perte de masse musculaire).

Une mesure de la circonférence de membres corporels est également indiquée pour constater une diminution du volume lors de suivis d'un régime diététique et lors de traitements anti-cellulites lors desquels un affinement du membre est attendu.

Une mesure de la circonférence de membres corporels est également indiquée pour constater une modification du volume lors d'un entraînement dans le cadre d'un programme de musculation où une augmentation du volume musculaire est attendue ou espérée.

Il est bien entendu que toute autre état ou pathologie entraînant une variation de la circonférence au niveau d'un membre corporel fait partie du cadre de la présente invention.

Par ailleurs, les Directives de l'Institut National d'Assurance Maladie-Invalidité (INAMI) recommandent aux praticiens (médecins, kinésithérapeutes, ...) d'effectuer des mesures tous les 4 cm le long du membre corporel présentant un gonflement ou souffrant d'une diminution du volume musculaire. Un nombre de mesures conséquent doit donc être effectué et il convient ainsi de disposer d'un outil de mesure qui, une fois positionné le long d'un membre corporel permette de relever ou d'effectuer des mesures à intervalles réguliers sur ce membre corporel de façon fiable, rapide, précise et reproductible.

Un dispositif de mesure de lymphoedème est par exemple connu du document GB2452256 qui divulgue un dispositif de mesure comprenant une règle télescopique (extensible) formée par une succession de segments, deux segments successifs présentant une section moindre depuis une extrémité proximale jusqu'à une extrémité distale de ladite règle graduée. En outre, ce dispositif comprend une sangle distale et une sangle proximale destinées l'une et l'autre à fixer le dispositif aux extrémités d'un membre corporel ou du moins en deux zones distinctes d'un membre corporel de telle sorte que la régie télescopique soit placée le long du membre où doivent être prises les mesures. Selon le dispositif décrit dans ce document antérieur, (es mesures de la circonférence peuvent être réalisées à l'aide d'une bande coulissante graduée qui peut être déplacée le long des segments de la règle télescopique graduée par l'intermédiaire d'un coulisseau. Un enroulement de cette bande coulissante autour d'une portion d'un membre corporel permet de définir la circonférence de ladite portion considérée à un endroit précis sur le membre corporel, cet endroit étant défini grâce à la graduation de la règle télescopique graduée.

Malheureusement, un tel dispositif de mesure de la circonférence de membres corporels comprenant une règle télescopique implique que les segments qui composent cette dernière doivent être suffisamment rigides de façon à pouvoir s'emboiter les uns dans les autres ou les uns sur les autres. Une telle rigidité, qui est obligatoire selon le dispositif du document GB2452256, rend difficile le placement d'un tel dispositif de mesure le long de tous les membres corporels et selon toutes les pathologies en assurant en contact étroit, c'est-à-dire contre la peau, en surface et tout le long du membre corporel de telle sorte que les mesures soient précises quelque soit la situation rencontrée. En effet, la rigidité de la règle graduée rend difficile son placement et son application contre et le long d'un membre corporel en suivant ce membre corporel selon ses éventuels courbures (coude, genoux, ...) et/ou protubérances et/ou gonflements, lesquels sont par exemple dus soit à la morphologie même du membre considéré, soit à la présence d'un oedème.

En outre, puisque les différents segments de la règle présentent des sections variables, c'est-à-dire des segments successifs dont la largeur d'un segment à l'autre diminue depuis une extrémité proximale jusqu'à une extrémité distale de ladite règle, le coulisseau permettant de déplacer la bande coulissante graduée doit impérativement présenter une ouverture ou orifice de passage de la coulisse pouvant s'engager sur le plus large de ces segments. Il en résulte que lorsque le coulisseau est positionné au niveau d'un segment présentant une largeur moindre que celle du plus large des segments, un certain « jeu » ou « flottement » entre ledit coulisseau et ledit segment de ladite règle graduée est observé, ce qui rend toute mesure approximative et non précise. En effet, un tel « jeu » ou « flottement » peut entraîner des imprécisions de mesures de l'ordre du centimètre à quelques centimètres, le coulisseau ayant tendance à présenter une position inclinée par rapport à l'axe défini par ladite règle graduée, ce qui implique inévitablement une imprécision de la mesure effectuée. Or, lorsque la décision de traiter ou non une pathologie tel qu'un lymphoedème repose sur une faible variation de Sa circonférence d'un membre corporel de l'ordre de 2 cm, une telle imprécision de l'ordre du centimètre est tout à fait inacceptable.

Par ailleurs, une règle télescopique présente forcément une limitation en termes de longueur. En effet, pour que le dispositif de mesure conserve une taille raisonnable, le nombre de segments télescopiques ne doit pas être excessif, ce qui conduirait à de trop nombreux emboîtements ou à de trop nombreuses superpositions qui, finalement, donneraient lieu à un dispositif volumineux et peu maniable. Aussi, si le dispositif doit pouvoir être apposé le long d'un membre corporel relativement long, par exemple le long d'un membre inférieur tel qu'une jambe, ceci impliquerait que le premier segment de la règle devrait être suffisamment large que pour pouvoir accueillir les autres segments télescopiques présentant une moindre taille ou largeur. Une telle largeur du premier segment serait problématique dès lors qu'elle pourrait empêcher un positionnement correct du dispositif de mesure le long d'un membre corporel.

L'invention a pour but de pallier les inconvénients de l'état de la technique en procurant un dispositif de mesure de la circonférence de membres corporels qui permette de réaliser des mesures rapides, précises, fiables et reproductibles quel que soit le membre corporel considéré, que ce dernier soit court ou long, présente des courbures et/ou des protubérances ou non et à n'importe quel endroit le long du membre corporel considéré. En outre l'invention a également pour objectif de procurer un dispositif de mesure qui présente une taille raisonnable, qui soit maniable, léger et peu encombrant pour par exemple être facilement rangé dans la poche d'un vêtement, ce qui constitue d'ailleurs un avantage certain dans le domaine médical où les professionnels du domaine de la santé, se déplaçant de chambres en chambres ou d'un cabinet à l'autre pour voir leurs patients, rangent fréquemment leur matériel dans les poches de leur blouse de travail.

Pour résoudre ces problèmes de l'état de la technique, il est prévu, suivant l'invention, un dispositif de mesure itérative de la circonférence d'un objet, en particulier d'un membre corporel, tel qu'indiqué au début, caractérisé en ce que :
- ladite coulisse est une coulisse souple présentant une section constante sur toute sa longueur,
- ledit orifice de passage est délimité par quatre parois dont deux au moins sont parallèles l'une à l'autre et forment chacune une paroi de guidage du coulisseau le long de la coulisse de façon à inscrire ledit deuxième élément de mesure gradué longitudinal dans un plan perpendiculaire à ladite première direction longitudinale,
- ladite sangle proximale est reliée à la coulisse ou à un coulisseau coopérant avec ladite coulisse autre que celui auquel est relié ledit deuxième
   élément de mesure gradué, ladite sangle proximale étant détachable ou non,
- ladite sangle distale est reliée à la coulisse ou à un coulisseau coopérant avec ladite coulisse autre que celui auquel est relié ledit deuxième élément de mesure gradué, ladite sangle distale étant détachable ou non, et en ce que
- ladite sangle distale et ladite sangle proximale sont des bandes longitudinales graduées ou non présentant une première et une deuxième extrémité munies chacune d'un moyen de fermeture et présentant une position ouverte et une position fermée, ladite position ouverte étant une position où lesdites première et deuxième extrémités sont espacées l'une de l'autre alors que ladite position fermée est une position dans laquelle ladite première extrémité de ladite bande longitudinale est rabattue sur ladite deuxième extrémité.

Un tel dispositif tel que préconisé suivant l'invention est particulièrement avantageux et permet de mesurer la circonférence de n'importe quel membre corporel à n'importe quel endroit sur ce membre, de façon rapide, précise, reproductible et fiable, que ce membre corporel présente des courbures et/ou des protubérances ou non.

En effet, selon la présente invention, une mesure précise de la circonférence d'un membre corporel peut être obtenue en toute circonstance puisque ledit au moins un coulisseau est agencé de telle sorte qu'il suive étroitement et perpendiculairement le premier élément de mesure gradué, lequel définit une coulisse présentant une section constante. Ceci est rendu possible par le fait que l'orifice de passage du coulisseau est délimité par quatre parois dont deux au moins sont parallèles l'une à l'autre et forment chacune une paroi de guidage du coulisseau le long de la coulisse de façon à inscrire ledit deuxième élément de mesure gradué longitudinal dans un plan perpendiculaire à ladite première direction longitudinale. La coulisse peut alors agir en tant que véritable guide (pilote) du coulisseau qui, lui-même, guide alors le deuxième élément de mesure étant par conséquent également parfaitement perpendiculaire par rapport au premier élément de mesure gradué (c'est-à-dire par rapport à la coulisse), le long duquel il peut se déplacer longitudinalement en conservant cette perpendicularité en permanence.

En outre, comme le premier élément de mesure gradué (ou coulisse) est formé dans un matériau souple, il peut parfaitement, sur l'ensemble de sa longueur, être apposé le long de membres corporels, directement contre la peau, même si ces membres corporels présentent des protubérances et/ou des creux. Par ailleurs, puisque le premier élément de mesure gradué est formé et fabriqué dans un matériau souple, il peut être facilement enroulé et/ou plié, ce qui permet d'en minimiser la taille et en facilite le rangement, le transport et la manipulation.

Par les termes « souple », « coulisse souple » ou « coulisse formée dans un matériau souple », on entend, au sens de la présente invention, une coulisse dont la souplesse lui permet de suivre, étroitement et par contact, la surface de l'objet ou du membre corporel dont la circonférence doit être mesurée, cette souplesse permettant par ailleurs un pliage, une courbure ou un enroulage de la coulisse. Cependant, au sens de la présente invention, cette souplesse ne doit pas être excessive pour ne pas que la largeur de la coulisse soit flexible et empêche la coulisse de rester sensiblement plane. Par exemple, la « souplesse » au sens de la présente invention ne peut pas être liée à une certaine élasticité qui tendrait à permettre une déformation de la coulisse lors de son placement le long d'un membre corporel.

Selon la présente invention, des mesures précises, fiables et reproductibles de la circonférence de membres corporels peuvent donc être réalisées dans toute circonstance, quel que soit le membre corporel considéré ou l'endroit du membre corporel considéré puisque le dispositif de mesure suivant l'invention permet, de par la souplesse du premier élément de mesure gradué de suivre parfaitement la surface du membre corporel mais aussi d'assurer la perpendicularité du deuxième élément de mesure gradué par rapport au premier élément de mesure gradué de part les propriétés du coulisseau dont l'orifice de passage de coulisse est délimité par quatre parois dont deux au moins sont parallèles l'une à l'autre et forment chacune une paroi de guidage du coulisseau le long de la coulisse de façon à inscrire ledit deuxième élément de mesure gradué longitudinal dans un plan perpendiculaire à ladite première direction longitudinale. De telles mesures à la fois précises, fiables et reproductibles de la circonférence de membres corporels sont indispensables afin d'éliminer toute marge d'erreur qui pourrait fausser les diagnostics et remettre en cause te principe même de la mesure de la circonférence de membres corporels comme outil décisionnel avant d'entamer ou non un traitement.

De préférence, selon le dispositif de mesure suivant l'invention, ladite coulisse et/ou ledit deuxième élément de mesure gradué est enroulable. Par exemple, ladite coulisse et/ou ledit deuxième élément de mesure gradué peut se présenter sous forme d'un mètre ruban tels que ceux utilisés dans le domaine de la couture et qui sont généralement formés dans un matériau en plastique souple ou dans du papier présentant une forte résistance à la traction, ce qui permet d'enrouler ces mètres rubans soit manuellement soit automatiquement à l'aide d'un système d'enroulement automatique comprenant par exemple un ressort de rappel. Un enroulement de la coulisse et/ou dudit deuxième élément de mesure gradué a pour avantage de minimiser la taille du dispositif de mesure lorsqu'il n'est pas utilisé, ce qui permet de le ranger sous une forme peu encombrante et de le transporter aisément. En outre, le fait que la coulisse et/ou ledit deuxième élément de mesure gradué puisse être enroulée permet à l'opérateur de n'en dérouler que la portion dont il a réellement besoin lors de la prise d'une mesure, la portion non utilisée de la coulisse et/ou dudit deuxième élément de mesure gradué restant enroulée, étant peu encombrante et n'entravant pas les mouvements de l'opérateur.

De préférence, selon le dispositif de mesure suivant l'invention, ladite coulisse et/ou ledit deuxième élément de mesure gradué est pliable, par exemple en forme de S. Les mêmes avantages que ceux mentionnés ci-dessus sont d'application lorsqu'un tel pliage de la coulisse est réalisé.

Avantageusement, le dispositif de mesure suivant l'invention comprend une pluralité de coulisseaux. Selon l'invention un nombre illimité de coulisseaux peuvent être ajoutés sur la coulisse. Lorsque plusieurs coulisseaux sont disponibles sur la coulisse, l'opérateur ou plusieurs opérateurs peuvent effectuer plusieurs mesures simultanément, ce qui permet de gagner du temps, notamment lorsqu'il convient d'effectuer des mesures ponctuelles selon un intervalle de quelques centimètres sur un segment corporel présentant une longueur relativement importante, par exemple une jambe. Eventuellement, des sangles additionnelles peuvent également être reliées aux coulisseaux si le positionnement de la coulisse requiert plus de deux points de fixation le long du membre corporel.

Selon le dispositif de mesure suivant l'invention, ladite sangle proximale est reliée à la coulisse ou à un coulisseau autre que celui auquel est relié ledit deuxième élément de mesure gradué, ladite sangle proximale étant détachable ou non. Lorsque la sangle proximale est reliée à la coulisse, sa position est fixe tandis que, avantageusement, lorsqu'elle est reliée à un coulisseau autre que celui auquel est relié ledit deuxième élément de mesure gradué, sa position est variable, ce qui permet de choisir précisément l'endroit ou zone de sa fixation sur le membre corporel considéré. Pouvoir détacher ladite sangle proximale de la coulisse ou du coulisseau permet de minimiser la taille du dispositif de mesure lorsqu'il n'est pas utilisé mais aussi de changer de sangle proximale si, par exemple, elle doit présenter une longueur ou une largeur moindre ou plus importante, ce qui est fonction du membre corporel considéré.

Avantageusement, selon le dispositif de mesure suivant l'invention, ledit orifice de passage dudit coulisseau présente une section similaire à la section de la coulisse. Si les sections de la coulisse et de l'orifice de passage du coulisseau sont sensiblement similaires, une perpendicularité du deuxième élément de mesure dans un plan perpendiculaire à ladite première direction longitudinale est d'autant mieux respectée puisque des sections similaires impliquent que les quatre parois délimitant l'orifice de passage du coulisseau sont en contact étroit avec la coulisse.

De préférence, selon le dispositif de mesure suivant l'invention, ladite coulisse est une coulisse en un matériau souple comme par exemple du plastique souple ou du papier présentant une résistance suffisante à la traction.

De préférence, suivant l'invention, lesdits premier et/ou deuxième éléments de mesure ainsi que lesdites sangles distales et/ou proximales sont réalisées dans un matériau de type toile plastifiée.

Avantageusement, selon le dispositif de mesure suivant l'invention, ledit deuxième élément de mesure relié au coulisseau est détachable. Par exemple, le deuxième élément de mesure peut comprendre un moyen de fixation sous forme d'un organe mâle d'un système de fixation sur lequel se fixe un organe femelle situé sur le coulisseau. Il pourrait également s'agir d'un moyen de fixation de type Velcro® ou de tout autre type de moyen de fixation adéquat. Pouvoir détacher le deuxième élément de mesure du coulisseau permet de minimiser la taille du dispositif de mesure lorsqu'il n'est pas utilisé mais aussi de changer d'élément de mesure si, par exemple, cet élément de mesure doit présenter une longueur ou une largeur moindre ou plus importante, ce qui est fonction du membre corporel considéré.

Selon le dispositif de mesure suivant l'invention, ladite sangle distale est reliée à la coulisse ou à un coulisseau autre que celui auquel est relié ledit deuxième élément de mesure gradué, ladite sangle distale étant détachable ou non. Lorsque la sangle distale est reliée à la coulisse, sa position est fixe tandis que, avantageusement, lorsqu'elle est reliée à un coulisseau autre que celui auquel est relié ledit deuxième élément de mesure gradué, sa position est variable, ce qui permet de choisir précisément l'endroit ou zone de sa fixation sur le membre corporel considéré. Pouvoir détacher ladite sangle distale de la coulisse ou du coulisseau permet de minimiser la taille du dispositif de mesure lorsqu'il n'est pas utilisé mais aussi de changer de sangle distale si, par exemple, elle doit présenter une longueur ou une largeur moindre ou plus importante, ce qui est fonction du membre corporel considéré.

Par exemple, selon l'invention, ladite sangle proximale et/ou ladite sangle distale est un collier de type Colson® réutilisable, c'est-à-dire qui peut être fermé puis ouvert. H pourrait également s'agir d'un collier de type Colson® à usage unique, c'est-à-dire uniquement prévu pour être fermé. Dans ce cas, si le collier est à usage unique, il conviendra de le couper afin de détacher le dispositif de mesure placé autour de l'objet dont la circonférence a été mesurée.

Selon le dispositif de mesure suivant l'invention, ladite sangle distale et ladite sangle proximale est une bande longitudinale graduée ou non présentant une première et une deuxième extrémité munies chacune d'un moyen de fermeture et présentant une position ouverte et une position fermée, ladite position ouverte étant une position où lesdites première et deuxième extrémités sont espacées l'une de l'autre alors que ladite position fermée est une position dans laquelle ladite première extrémité de ladite bande longitudinale est rabattue sur ladite deuxième extrémité.

De préférence, selon le dispositif de mesure suivant l'invention, ledit moyen de fermeture est choisi dans le groupe constitué d'une fermeture à bouton-pression, à bouton-poussoir ou par moyen auto-agrippant, par exemple de type Velcro®. Un tel moyen de fermeture est particulièrement pratique puisqu'il peut être ouvert et fermé rapidement d'un simple mouvement de traction ou de pression, il est bien entendu que tout autre type de moyen de fermeture adéquat fait également l'objet de ta présente invention.

Avantageusement, selon l'invention, l'extrémité de la coulisse au niveau de laquelle est reliée ladite sangle distale comprend en outre une portion longitudinale supplémentaire graduée ou non s'étendant en prolongement de ladite coulisse au-delà de la graduation zéro de cette dernière. La présence d'une telle portion supplémentaire permet un placement de la sangle distale, par l'intermédiaire ou non d'un coulisseau, en amont de ta graduation zéro de telle sorte que ladite sangle distale n'empêche pas une visualisation précise de la graduation zéro. En outre, cette portion supplémentaire peut permettre, en amont de la graduation zéro, le placement éventuel de sangles additionnelles ou de tout autre éléments de fixation et/ou d'éléments de mesure additionnels. Cette portion supplémentaire peut être elle-même prolongée par un embout amovible ou non constituant une butée empêchant les coulisseaux de « quitter » ladite coulisse prolongée par cette portion supplémentaire.

Avantageusement, selon l'invention, le dispositif de mesure comprend en outre un système de blocage de la sangle distale et/ou de la sangle proximale. La présence de tels systèmes de blocage (ou systèmes de frein) permet d'améliorer la précision des mesures effectuées à intervalles réguliers en assurant le maintien en place du premier élément de mesure le long duquel peuvent se déplacer une série de coulisseaux reliés chacun à un deuxième élément de mesure gradué longitudinal. Il est bien entendu que tout type de système de blocage permettant de réaliser un tel blocage de la sangle distale et/ou proximale fait partie de la présente invention.

De préférence, suivant l'invention, lorsque ledit deuxième élément de mesure gradué est associé à un dispositif de type enrouleur afin d'y être rangé, le poids de ce dernier est de l'ordre de 10 à 50 grammes, de préférence de l'ordre de 25 à 30 grammes, préférentiellement de l'ordre de 20 grammes. Il a en effet été déterminé qu'un tel poids du dispositif de type enrouleur est adéquat dès lors que, sous la force de gravité, il exerce une traction constante permettant d'assurer une précision des mesures effectuées. En effet, la force de traction ainsi exercée sur ledit deuxième élément de mesure gradué assure que ce dernier n'est pas déformé mais aussi qu'il se positionne correctement par rapport au premier élément de mesure. Les risques d'erreur sont par conséquent minimisés lors de la mesure de la circonférence d'un objet.

Avantageusement, suivant l'invention, lorsque des coulisseaux sont présents le long dudit premier élément de mesure et lorsqu'il est prévu que ledit premier élément de mesure soit enroulé dans un enrouleur pour y être rangé, tant ledit premier élément de mesure que lesdits coulisseaux sont agencés pour être rangés simultanément dans le même enrouleur.

De préférence, suivant l'invention, ledit deuxième élément de mesure est muni, à son extrémité non reliée au premier élément de mesure, d'un embout amovible ou non comprenant une première paire de saillies s'étendant vers le haut dans une direction perpendiculaire au plan formé par ledit deuxième élément de mesure gradué et/ou une deuxième paire de saillies s'étendant vers l'extérieur sensiblement dans le même plan que celui formé par ledit deuxième élément de mesure gradué. Ceci est particulièrement avantageux dès lors que, d'une part, ladite première paire de saillies forme un guide pour la partie dudit deuxième élément de mesure s'y engageant lors de la mesure de la circonférence d'un objet. En effet, un tel guide permet au deuxième élément de mesure de prendre appui latéralement contre lesdites saillies, ce qui permet d'assurer une précision des mesures effectués en évitant les déplacements latéraux de la partie dudit deuxième élément de mesure se superposant sur une autre partie dudit deuxième élément de mesure lors d'une prise de mesure de la circonférence d'un objet. D'autre part, ladite deuxième paire de saillies forme un repère correspondant à la graduation zéro, ce qui permet, lors de la prise de mesure, de lire facilement et précisément la valeur de la circonférence de l'objet mesuré.

Selon un mode de réalisation suivant l'invention, ladite première paire de saillies peut présenter des extrémités libres courbes orientées vers ledit deuxième élément de mesure gradué. Ceci permet d'assurer le maintien de la partie dudit deuxième élément de mesure se superposant sur une autre partie dudit deuxième élément de mesure lors d'une prise de mesure de la circonférence d'un objet.

D'autres formes de réalisation du dispositif de mesure de la circonférence de segments corporels suivant l'invention sont indiquées dans les revendications annexées.

L'invention a aussi pour objet un kit pour former un dispositif de mesure selon l'invention, pour la mesure de la circonférence d'un objet, en particulier d'un membre corporel, ledit kit comprenant :
- au moins un premier élément de mesure gradué présentant une première direction longitudinale et définissant une coulisse souple présentant une section constante sur toute sa longueur et étant agencée pour être apposée le long d'un segment corporel,
- une pluralité de deuxièmes éléments de mesure gradués,
- une pluralité de sangles distales agencées pour être inscrites chacune dans un plan sensiblement perpendiculaire à ladite direction longitudinale et agencées pour être reliées à la coulisse ou à un coulisseau autre que celui auquel est relié ledit deuxième élément de mesure gradué, lesdites sangles distales étant détachables ou non, lesdites sangles distales étant des bandes longitudinales graduées ou non présentant une première et une deuxième extrémité munies chacune d'un moyen de fermeture et présentant une position ouverte et une position fermée, ladite position ouverte étant une position où lesdites première et deuxième extrémités sont espacées l'une de l'autre alors que ladite position fermée est une position dans laquelle ladite première extrémité de ladite bande longitudinale est rabattue sur ladite deuxième extrémité,
- une pluralité de sangles proximales agencées pour être inscrites chacune dans un plan sensiblement perpendiculaire à ladite direction longitudinale et agencées pour être reliées à la coulisse ou à un coulisseau autre que celui auquel est relié ledit deuxième élément de mesure gradué, lesdites sangles proximales étant détachables ou non, lesdites sangles proximales étant des bandes longitudinales graduées ou non présentant une première et une deuxième extrémité munies chacune d'un moyen de fermeture et présentant une position ouverte et une position fermée, ladite position ouverte étant une position où lesdites première et deuxième extrémités sont espacées l'une de l'autre alors que ladite position fermée est une position dans laquelle ladite première extrémité de ladite bande longitudinale est rabattue sur ladite deuxième extrémité, et
- une pluralité de coulisseaux agencés pour coopérer avec ladite coulisse et présentant un orifice de passage de coulisse délimité par quatre parois dont deux au moins sont parallèles l'une à l'autre et forment chacune une paroi de guidage du coulisseau le long de la coulisse de façon à inscrire au moins un deuxième élément de mesure gradué longitudinal dans un plan perpendiculaire à ladite première direction longitudinale dudit premier élément de mesure gradué, lesdits coulisseaux étant agencés pour être positionnés entre ladite pluralité de sangles distales et ladite pluralité de sangles proximales.

Un tel kit pour la mesure de la circonférence de membres corporels comprend peu d'éléments qui peuvent être facilement et rapidement assemblés afin de disposer du dispositif de mesure de la circonférence de membres corporels tel que décrit ci-avant. Ce kit contenant tous les éléments essentiels de la présente invention est de petite taille et peut tenir aisément dans une boite ayant un format inférieur ou égal au format d'une poche d'un vêtement de travail (par exemple un tablier de laboratoire).

D'autres formes de réalisation du kit pour la mesure de la circonférence de segments corporels suivant l'invention sont indiquées dans les revendications annexées.

D'autres caractéristiques, détails et avantages de l'invention ressortiront de la description donnée ci-après, à titre non limitatif et en faisant référence aux dessins annexés.

La figure 1a est une vue schématique d'un premier mode de réalisation d'un dispositif de mesure de la circonférence de segments corporels sous sa forme montée selon l'invention. La figure 1b est une vue de détail et de côté d'un coulisseau suivant l'invention, selon la zone indiquée à la figure 1 a par le cercle en traits interrompus.

La figure 2 est une vue schématique d'un autre mode de réalisation d'un dispositif de mesure de la circonférence de segments corporels sous sa forme montée selon l'invention.

La figure 3 est une vue schématique d'un exemple d'un kit pour la mesure de la circonférence de segments corporels suivant l'invention.

La figure 4 illustre un embout pouvant être présent à une extrémité d'un élément de mesure gradué.

Sur les figures, les éléments identiques ou analogues portent les mêmes références.

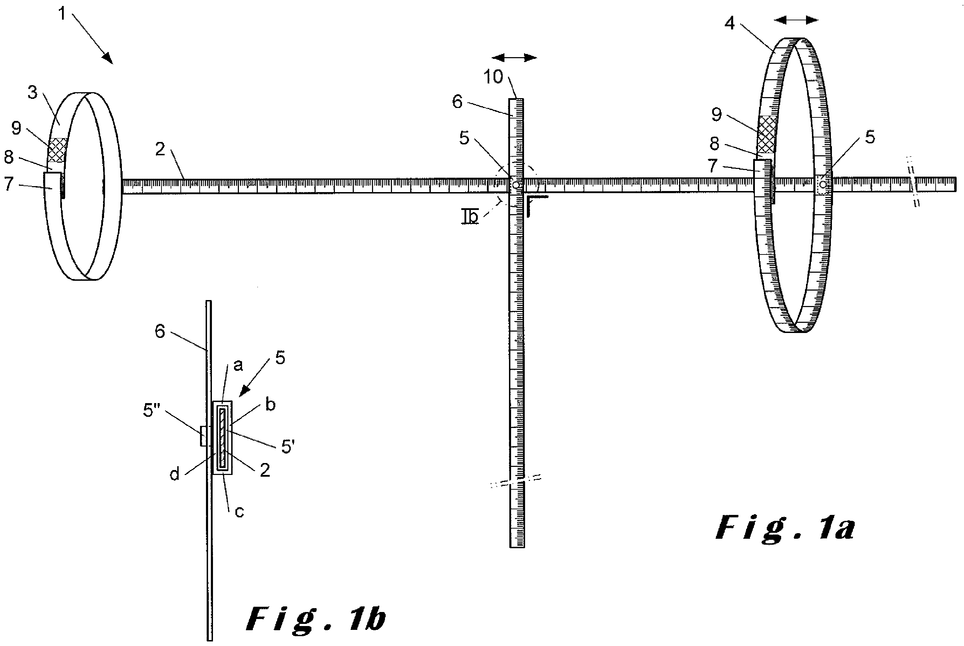

La figure 1 a illustre un premier mode de réalisation d'un dispositif de mesure 1 de la circonférence de membres corporels sous sa forme montée. Ce dispositif de mesure 1 comprend un premier élément de mesure gradué longitudinal ou coulisse 2. Il peut par exemple s'agir d'un mètre ruban en plastique souple ou en papier résistant aux forces de traction tel que ceux utilisés dans le domaine de la couture ou de tout autre matériau présentant des propriétés lui permettant de suivre étroitement et par contact sur toute sa longueur un objet ou un membre corporel. Cette coulisse 2 forme un guide pour des coulisseaux 5 qui présente un orifice de passage (tel que représenté à la figure 1b sous le numéro 5') de ladite coulisse 2. Un deuxième élément de mesure gradué longitudinal 6 est relié à un coulisseau 5, par exemple par emboîtement sur un moyen de fixation (tel que représenté à la figure 1b sous ie numéro 5") présent sur ledit coulisseau 5. Le moyen de fixation 5" (voir figure 1 b) permet d'ajuster ledit deuxième élément de mesure gradué longitudinal 6 perpendiculairement par rapport à ladite coulisse 2.

Le dispositif de mesure 1 comprend en outre au moins deux sangles 3, 4 positionnées de part et d'autre dudit coulisseau 5 auquel est relié ledit deuxième élément de mesure gradué 6, il s'agit plus particulièrement d'une sangle distale 3 et d'une sangle proximale 4 qui sont chacune reliée à ladite coulisse 2. Selon ce premier mode de réalisation, la sangle distale 3 est solidaire de ladite coulisse 2 sur laquelle elle est par exemple collée ou cousue tandis que la sangle proximale 4 est reliée à un coulisseau 5 de configuration identique à celui auquel est relié ledit deuxième élément de mesure gradué 6. La sangle distale 3 est donc fixe alors que la sangle proximale 4 ainsi que ledit deuxième élément de mesure gradué 6 sont mobiles et peuvent être déplacés longitudinalement le long de la coulisse 2 vers la droite ou vers la gauche (comme indiqué par les doubles flèches). Chacune des sangles 3, 4 comprend en outre une première extrémité 7 et une deuxième extrémité 8, ladite première extrémité 7 étant rabattue sur ladite deuxième extrémité 8 afin de refermer lesdites sangles 3, 4 suite à leur positionnement autour d'un membre corporel. Comme illustré, plusieurs zones de fixation 9 sont prévues sur les sangles 3, 4 de telle sorte que ces dernières puissent être fixée autour de membres corporels présentant des circonférences différentes.

Une fois les deux sangles 3, 4 positionnées correctement de telle façon que ladite coulisse 2 soit apposée à la surface du membre corporel considéré, un opérateur peut effectuer autant de mesures qu'il le souhaite selon n'importe quel intervalle, par exemple tous les deux centimètres en déplaçant simplement, le long de la coulisse 2, le coulisseau 5 auquel est relié perpendiculairement ledit deuxième élément de mesure gradué 6. Lorsque l'opérateur a déplacé ledit deuxième élément de mesure gradué 6 jusqu'à l'endroit où il souhaite effectuer une mesure de la circonférence, cet endroit correspondant à l'une des graduations de la coulisse 2, il lui suffit d'enrouler le deuxième élément de mesure 6 autour du membre corporel pour en déterminer la circonférence correspondant à la mesure indiquée par la graduation du deuxième élément de mesure gradué 6 se trouvant en face de la graduation zéro 10 de ce même deuxième élément de mesure gradué 6. Selon ce premier mode de réalisation, seule la sangle proximale 4 comprend des graduations. Il est bien entendu que la sangle distale pourrait également comprendre des graduations ou que, dans une forme de réalisation alternative suivant l'invention, chacune des sangles 3, 4 soient dépourvues de graduations.

La figure 1b illustre, en détail et vu de côté, un coulisseau 5 suivant l'invention. Le coulisseau 5 présente un orifice de passage 5' délimité par quatre parois a, b, c et d, lesquelles forment chacune une paroi de guidage du coulisseau 5 le long de la coulisse 2 de façon à inscrire le deuxième élément de mesure gradué longitudinal 6 dans un plan perpendiculaire à la direction longitudinale de la coulisse 2.

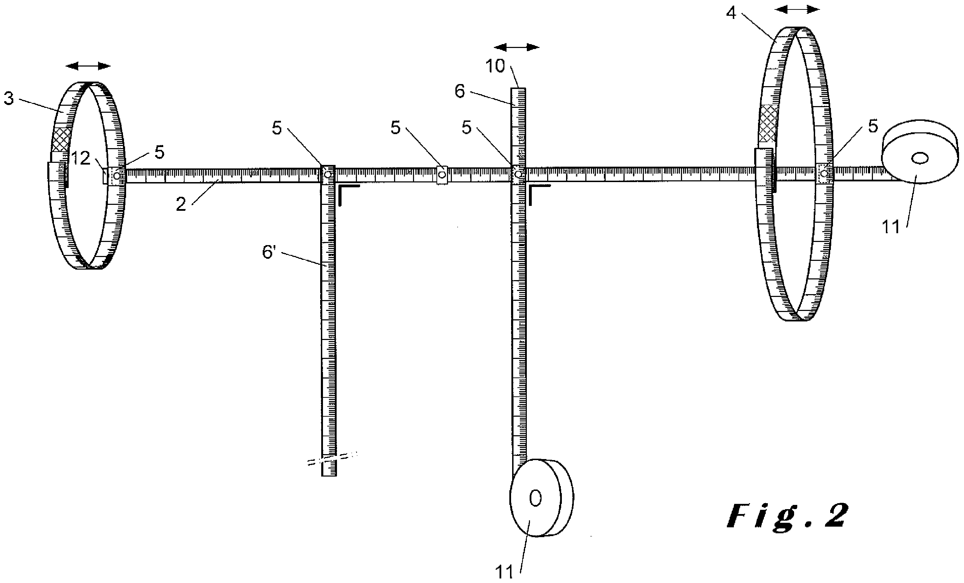

La figure 2 illustre un autre mode de réalisation d'un dispositif de mesure de la circonférence de segments corporels 1 sous sa forme montée. Cette figure reprend les mêmes éléments que ceux décrits à la figure 1. Cependant, selon cet autre mode de réalisation, le dispositif de mesure 1 suivant l'invention comprend trois coulisseaux 5 additionnels, l'un auquel est reliée la sangle distale 3, l'un auquel est relié un deuxième élément de mesure gradué additionnel 6' et l'un auquel n'est relié aucun élément. Comme illustré, il est donc possible que les deux sangles 3, 4 soient toutes deux reliées à des coulisseaux distincts 5 permettant leur déplacement vers la droite ou vers la gauche (comme indiqué par les doubles flèches) longitudinalement et perpendiculairement le long de la coulisse 2. La figure 2 illustre également que les deuxièmes éléments de mesure gradués 6, 6' peuvent indifféremment présenter leur extrémité comprenant la graduation zéro 10 soit le long du bord supérieur de la coulisse 2 (cas du deuxième élément de mesure gradué 6'), soit au-delà du bord supérieur de la coulisse 2 (cas du deuxième élément de mesure gradué 6). Le coulisseau 5 auquel n'est relié aucun élément pourrait permettre par exemple d'y relier une sangle additionnelle ou encore un deuxième élément de mesure gradué additionnel. Selon ce second mode de réalisation, le deuxième élément de mesure gradué 6 ainsi que la coulisse 2 sont tous deux des éléments de mesure enroulables et rétractables à l'aide d'un système d'enroulement automatique ou manuel 11 comprenant par exemple un ressort de rappel et un frein actionnable manuellement. Il est bien entendu que le deuxième élément de mesure gradué 6' pourrait également être relié à un tel dispositif d'enroulage 11.

Ce mode de réalisation selon la figure 2 est particulièrement avantageux puisque tous les éléments reliés à un coulisseau peuvent en être désolidarisés de telle sorte que, après démontage du dispositif de mesure selon cet exemple, l'opérateur se retrouve avec deux sangles 3, 4, une coulisse 2 qui peut être enroulée dans un dispositif d'enroulage 11 et qui comprend cinq coulisseaux et deux deuxièmes éléments de mesure gradués, l'un 6 pouvant être enroulée dans un dispositif d'enroulage 11 et l'autre 6' pouvant par exemple être plié en forme de S. Afin que, suite à l'enroulement de la coulisse 2, les coulisseaux 5 coopérant avec cette dernière y restent positionnés, un embout 12 amovible ou non peut être prévu à l'extrémité de ladite coulisse 2 pour former une butée contre laquelle sont bloqués les coulisseaux 5 de telle sorte qu'ils ne peuvent pas « quitter » ladite coulisse 2. Le fait que cet embout 12 soit amovible permet, au besoin, l'ajout d'un coulisseau 5 ou d'un élément de mesure supplémentaire 6.

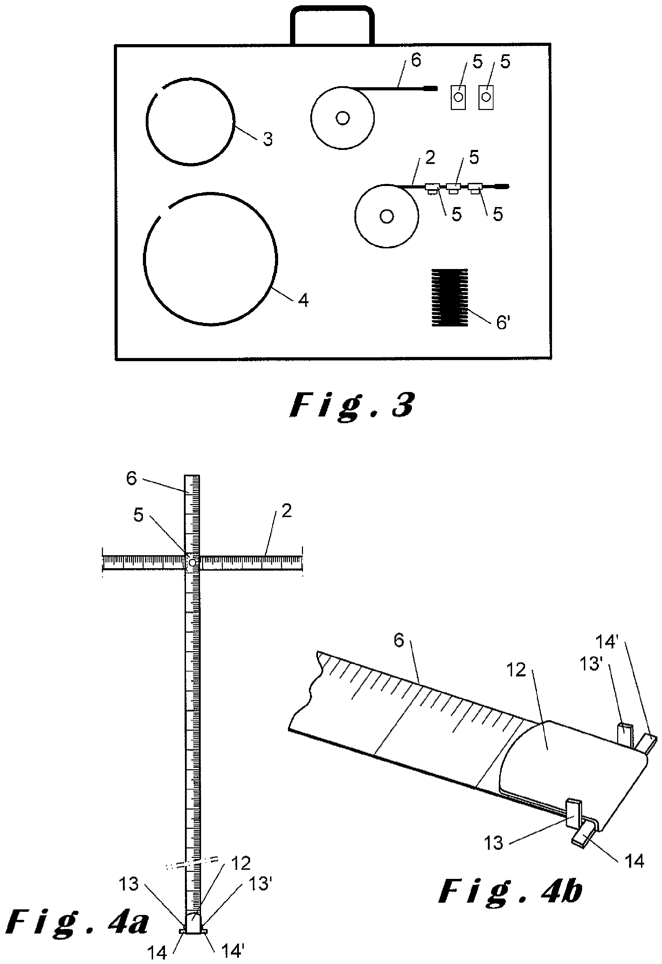

La figure 3 illustre un kit pour la mesure de la circonférence de segments corporels suivant l'invention, ce qui comprenant un premier élément de mesure gradué 2, une pluralité de coulisseaux 5 (certains positionnés sur la coulisse 2, d'autres non), deux deuxièmes éléments de mesure gradués 6, 6' (l'un plié sous forme de S et l'autre enroulé dans un dispositif d'enroulage) et deux sangles 3, 4. M est bien entendu que le kit selon l'invention n'est aucunement limité à cet exemple et qu'un kit qui comprendrait un élément donné en un autre nombre d'exemplaire fait également partie intégrante de la présente invention.

Les figures 4a et 4b illustrent un deuxième élément de mesure gradué 6 muni à son extrémité non reliée au premier élément de mesure gradué 2 d'un embout 12 amovible ou non comprenant une première paire de saillies 13, 13' s'étendant vers le haut dans une direction perpendiculaire au plan formé par ledit deuxième élément de mesure gradué 6 et une deuxième paire de saillies 14, 14' s'étendant vers l'extérieur sensiblement dans le même plan que celui formé par ledit deuxième élément de mesure gradué 6.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Dispositif de mesure (1) itérative de la circonférence d'un objet, en particulier d'un membre corporel, comprenant :
- un premier élément de mesure gradué longitudinal (2) présentant une première direction longitudinale, agencé pour être apposé le long dudit objet, en particulier le long dudit membre corporel et définissant une coulisse (2),
- une sangle distale (3) et une sangle proximale (4) inscrite chacune dans un plan sensiblement perpendiculaire à ladite direction longitudinale, et
- un coulisseau (5) coopérant avec ladite coulisse (2), relié à un deuxième élément de mesure gradué longitudinal (6), ledit coulisseau (5) présentant un orifice de passage de coulisse (5') et étant positionné entre ladite sangle distale (3) et ladite sangle proximale (4),
**caractérisé en ce que** :
- ladite coulisse (2) est une coulisse souple présentant une section constante sur toute sa longueur, -
- ledit orifice de passage (5') est délimité par quatre parois dont deux au moins sont parallèles l'une à l'autre et forment chacune une paroi de guidage du coulisseau (5) le long de la coulisse (2) de façon à inscrire ledit deuxième élément de mesure gradué longitudinal (6) dans un plan perpendiculaire à ladite première direction longitudinale,
- ladite sangle proximale (4) est reliée à la coulisse (2) ou à un coulisseau (5) coopérant avec ladite coulisse autre que celui auquel est relié ledit deuxième élément de mesure gradué (6), ladite sangle proximale (4) étant détachable ou non,
- ladite sangle distale (3) est reliée à la coulisse (2) ou à un coulisseau (5) coopérant avec ladite coulisse
autre que celui auquel est relié ledit deuxième élément de mesure gradué (6), ladite sangle distale (3) étant détachable ou non, et **en ce que**
- ladite sangle distale (3) et ladite sangle proximale (4) sont des bandes longitudinales graduées ou non présentant une première (7) et une deuxième (8) extrémité munies chacune d'un moyen de fermeture (9) et présentant une position ouverte et une position fermée, ladite position ouverte étant une position où lesdites première (7) et deuxième (8) extrémités sont espacées l'une de l'autre alors que ladite position fermée est une position dans laquelle ladite première extrémité (7) de ladite bande longitudinale est rabattue sur ladite deuxième extrémité (8).

2. Dispositif de mesure (1) selon la revendication 1, **caractérisé en ce que** ladite coulisse (2) et/ou ledit deuxième élément de mesure gradué (6) est enroulable.

3. Dispositif de mesure selon la revendication 1, **caractérisé en ce que** ladite coulisse (2) et/ou ledit deuxième élément de mesure gradué (6) est pliable, par exemple en forme de S.

4. Dispositif de mesure (1) selon l'une quelconque des revendications 1 à 3, **caractérisé en ce qu'**il comprend une pluralité de coulisseaux (5).

5. Dispositif de mesure (1) selon l'une quelconque des revendications 1 à 4, **caractérisé en ce que** ledit orifice de passage (5') dudit coulisseau (5) présente une section similaire à la section de la coulisse (2).

6. Dispositif de mesure (1) selon l'une quelconque des revendications 1 à 5, **caractérisé en ce que** ladite coulisse (2) est une coulisse en un matériau souple comme par exemple du plastique souple ou du papier présentant une résistance suffisante à la traction.

7. Dispositif de mesure (1) selon l'une quelconque des revendications 1 à 6, **caractérisé en ce que** ledit deuxième élément de mesure (6) relié au coulisseau (5) est détachable.

8. Dispositif de mesure (1) selon l'une quelconque des revendications 1 à 7, **caractérisé en ce que** ledit moyen de fermeture (9) est choisi dans le groupe constitué d'une fermeture à bouton-pression, à bouton-poussoir ou par moyen auto-agrippant, par exemple de type Velcro®.

9. Dispositif de mesure (1) selon l'un quelconque des revendications 1 à 8, **caractérisé en ce que** ledit deuxième élément de mesure (6) est muni, à son extrémité non reliée au premier élément de mesure (2), d'un embout (12) amovible ou non comprenant une première paire de saillies (13, 13') s'étendant vers le haut dans une direction perpendiculaire au plan formé par ledit deuxième élément de mesure gradué (6) et/ou une deuxième paire de saillies (14, 14') s'étendant vers l'extérieur sensiblement dans le même plan que celui formé par ledit deuxième élément de mesure gradué (6).

10. Kit pour former un dispositif de mesure selon l'une quelconque des revendications 1 à 9, pour la mesure de la circonférence d'un objet, en particulier d'un membre corporel, ledit kit comprenant :
- au moins un premier élément de mesure gradué (2) présentant une première direction longitudinale et définissant une coulisse (2) souple présentant une section constante sur toute sa longueur et étant agencée pour être apposée le long d'un segment corporel,
- une pluralité de deuxièmes éléments de mesure gradués (6),
- une pluralité de sangles distales (3) agencées pour être inscrites chacune dans un plan sensiblement perpendiculaire à ladite direction longitudinale et agencées pour être reliées à la coulisse (2) ou à un coulisseau (5) autre que celui auquel est relié ledit deuxième élément de mesure gradué (6), lesdites sangles distales (3) étant détachables ou non, lesdites sangles distales (3) étant des bandes longitudinales graduées ou non présentant une première (7) et une deuxième (8) extrémité munies chacune d'un moyen de fermeture (9) et présentant une position ouverte et une position fermée, ladite position ouverte étant une position où lesdites première (7) et deuxième (8) extrémités sont espacées l'une de l'autre alors que ladite position fermée est une position dans laquelle ladite première extrémité (7) de ladite bande longitudinale est rabattue sur ladite deuxième extrémité (8),
- une pluralité de sangles proximales (4) agencées pour être inscrites chacune dans un plan sensiblement perpendiculaire à ladite direction longitudinale et agencées pour être reliées à la coulisse (2) ou à un coulisseau (5) autre que celui auquel est relié ledit deuxième élément de mesure gradué (6), lesdites sangles proximales (4) étant détachables ou non, lesdites sangles proximales (4) étant des bandes longitudinales graduées ou non présentant une première (7) et une deuxième (8) extrémité munies chacune d'un moyen de fermeture (9) et présentant une position ouverte et une position fermée, ladite position ouverte étant une position où lesdites première (7) et deuxième (8) extrémités sont espacées l'une de l'autre alors que ladite position fermée est une position dans laquelle ladite première extrémité (7) de ladite bande longitudinale est rabattue sur ladite deuxième extrémité (8), et
- une pluralité de coulisseaux (5) agencés pour coopérer avec ladite coulisse (2) et présentant un orifice de passage de coulisse (5') délimité par quatre parois dont deux au moins sont parallèles l'une à l'autre et forment chacune une paroi de guidage du coulisseau (5) le long de la coulisse (2) de façon à inscrire au moins un deuxième élément de mesure gradué longitudinal (6) dans un plan perpendiculaire à ladite première direction longitudinale dudit premier élément de mesure gradué (2), lesdits coulisseaux (5) étant agencés pour être positionnés entre ladite pluralité de sangles distales (3) et ladite pluralité de sangles proximales (4).

## Patentansprüche

1. Iterative Messvorrichtung (1) des Umfangs eines Gegenstandes, insbesondere eines Körperteils, umfassend:
- ein erstes, längliches, graduiertes Messelement (2), das eine erste Längsrichtung aufweist, die angeordnet ist, um entlang des genannten Gegenstandes angeordnet zu sein, insbesondere entlang des genannten Körperteils, und eine Gleitschiene (2) definiert,
- einen distalen Gurt (3) und einen proximalen Gurt (4), die jeweils in einer Ebene angeordnet sind, die deutlich lotrecht zu der genannten Längsrichtung ist, und
- einen Gleitschlitten (5), der mit der genannten Gleitschiene (2) zusammenwirkt, mit dem zweiten länglichen graduierten Messelement (6) verbunden ist, wobei der genannten Gleitschlitten (5) eine Durchgangsöffnung der Gleitschiene (5') aufweist und zwischen dem genannten distalen Gurt (3) und dem genannten proximalen Gurt (4) positioniert ist,
**dadurch gekennzeichnet, dass**:
- die genannte Gleitschiene (2) eine bewegliche Gleitschiene ist, die einen über ihre gesamte Länge konstanten Querschnitt aufweist,
- die genannte Durchgangsöffnung (5') durch vier Wände begrenzt ist, von denen wenigstens zwei zueinander parallel sind und jeweils eine Führungswand des Schlittens (5) entlang der Gleitschiene (2) derart bilden, dass das genannte zweite längliche, graduierte Messelement (6) in einer Ebene angeordnet ist, die zu der genannten ersten Längsrichtung lotrecht ist,
- der genannte proximale Gurt (4) mit der Gleitschiene (2) oder einem Gleitschlitten (5) verbunden ist, der mit der genannten Gleitschiene zusammenwirkt und nicht der ist, mit dem das zweite graduierte Messelement (6) verbunden ist, wobei der genannte proximale Gurt (4) abnehmbar oder nicht abnehmbar ist,
- wobei der genannte distale Gurt (3) mit der Gleitschiene (2) oder einem Gleitschlitten (5) verbunden ist, der mit der genannten Gleitschiene zusammenwirkt, der nicht der ist, der mit dem genannten zweiten graduierten Messelement (6) verbunden ist, wobei der genannte distale Gurt (3) abnehmbar oder nicht abnehmbar ist, und dass
- der genannte distale Gurt (3) und der genannte proximale Gurt (4) graduierte oder nicht graduierte längliche Bänder sind, die ein erstes (7) und ein zweites (8) Ende aufweisen, die jeweils mit einem Verschlussmittel (9) versehen sind und eine offene Position und eine geschlossene Position aufweisen, wobei die genannte offene Position eine Position ist, in der die genannten ersten (7) und zweiten (8) Enden voneinander beabstandet sind, während die genannte geschlossene Position eine Position ist, in der das genannte erste Ende (7) des genannten länglichen Bandes auf dem genannten zweiten Ende (8) umgeschlagen ist.

2. Messvorrichtung (1) gemäß Anspruch 1, **dadurch gekennzeichnet, dass** die genannte Gleitschiene (2) und / oder das genannte zweite graduierte Messelement (6) einrollfähig ist / sind.

3. Messvorrichtung gemäß Anspruch 1, **dadurch gekennzeichnet, dass** die genannte Gleitschiene (2) und / oder das genannte zweite graduierte Messelement (6) faltbar, z. B. in S-Form, ist / sind.

4. Messvorrichtung (1) gemäß irgendeinem der Ansprüche 1 bis 3, **dadurch gekennzeichnet, dass** sie eine Vielzahl von Gleitschlitten (5) umfasst.

5. Messvorrichtung (1) gemäß irgendeinem der Ansprüche 1 bis 4, **dadurch gekennzeichnet, dass** die genannte Durchgangsöffnung (5') des genannten Gleitschlittens (5) einen ähnlichen Querschnitt aufweist wie den Querschnitt der Gleitschiene (2).

6. Messvorrichtung (1) gemäß irgendeinem der Ansprüche 1 bis 5, **dadurch gekennzeichnet, dass** die genannte Gleitschiene (2) eine Gleitschiene aus einem beweglichen Material ist, wie z. B. weichem Plastik oder Papier, das einen ausreichenden Zugwiderstand aufweist.

7. Messvorrichtung (1) gemäß irgendeinem der Ansprüche 1 bis 6, **dadurch gekennzeichnet, dass** das genannte zweite Messelement (6), das mit dem Gleitschlitten (5) verbunden ist, abnehmbar ist.

8. Messvorrichtung (1) gemäß irgendeinem der Ansprüche 1 bis 7, **dadurch gekennzeichnet, dass** das genannte Verschlussmittel (9) aus der Gruppe ausgewählt ist, die aus einem Verschluss per Druckknopf, einem Druckknopf oder durch ein Klettverschlussmittel, z. B. vom Typ Velcro®, besteht.

9. Messvorrichtung (1) gemäß irgendeinem der Ansprüche 1 bis 8, **dadurch gekennzeichnet, dass** das genannte zweite Messelement (6) an seinem nicht mit dem ersten Messelement (2) verbundenen Ende mit einer abnehmbaren oder nicht abnehmbaren Endabdeckung (12) versehen ist, die ein erstes Vorsprungspaar (13, 13'), das sich nach oben in einer Richtung erstreckt, die lotrecht zu der Ebene ist, die durch das genannte zweite graduierte Messelement (6) gebildet ist, und / oder ein zweites Vorsprungspaar (14, 14'), das sich nach außer deutlich in derselben Ebene erstreckt wie der, die durch das genannte zweite graduierte Messelement (6) gebildet ist, umfasst.

10. Kit zum Bilden einer Messvorrichtung gemäß irgendeinem der Ansprüche 1 bis 9 zum Messen des Umfangs eines Gegenstandes, insbesondere eines Körperteils, wobei das genannte Kit umfasst:
- wenigstens ein erstes graduiertes Messelement (2), das eine erste Längsrichtung aufweist und eine bewegliche Gleitschiene (2) definiert, die einen konstanten Querschnitt über ihre gesamte Länge aufweist und angeordnet ist, um entlang einem Körpersegment aufgebracht zu werden,
- eine Vielzahl von zweiten graduierten Messelementen (6),
- eine Vielzahl von distalen Gurten (3), die angeordnet sind, um jeweils in einer Ebene angeordnet zu sein, die deutlich lotrecht zu der genannten Längsebene und angeordnet sind, um mit der Gleitschiene (2) oder mit einem Gleitschlitten (5) verbunden zu sein, der nicht der ist, der mit dem genannten zweiten graduierten Messelement (6) verbunden ist, wobei die genannten distalen Gurte (3) abnehmbar oder nicht abnehmbar sind, wobei die genannten distalen Gurte (3) längliche, graduierte oder nicht graduierte Bänder sind, die ein erstes (7) und ein zweites (8) Ende aufweisen, das jeweils mit einem Verschlussmittel (9) versehen ist und eine offene Position und eine geschlossene Position aufweist, wobei die genannte offene Position eine Position ist, in der das genannte erste (7) und zweite (8) Ende voneinander beabstandet sind, wobei die genannte geschlossene Position eine Position ist, in der das genannte erste Ende (7) des genannten länglichen Bandes auf dem genannten zweiten Ende (8) umgeschlagen ist,
- eine Vielzahl von proximalen Gurten (4), die angeordnet sind, um jeweils in einer Ebene angeordnet zu sein, die deutlich lotrecht zu der genannten Längsrichtung ist, und angeordnet sind, um mit der Gleitschiene (2) oder einem Gleitschlitten (5) verbunden zu sein, der nicht der ist, der mit dem genannten zweiten graduierten Messelement (6) verbunden ist, wobei die genannten proximalen Gurte (4) abnehmbar oder nicht abnehmbar sind, wobei die genannten proximalen Gurte (4) längliche, graduierte oder nicht graduierte Bänder sind, die ein erstes (7) und ein zweites (8) Ende aufweisen, die jeweils mit einem Verschlussmittel (9) versehen sind und eine offene Position und eine geschlossene Position aufweisen, wobei die genannte offene Position eine Position ist, in der das genannte erste (7) und zweite (8) Ende voneinander beabstandet sind, während die genannte geschlossene Position eine Position ist, in der das genannte erste Ende (7) des genannten länglichen Bandes auf dem genannten zweiten Ende (8) umgeschlagen ist, und
- eine Vielzahl von Gleitschlitten (5), die angeordnet sind, um mit der genannten Gleitschiene (2) zusammenzuwirken und eine Durchgangsöffnung (5') der Gleitschiene aufweisen, die durch vier Wände begrenzt ist, von denen wenigstens zwei zueinander parallel sind und jeweils eine Führungswand des Gleitschlittens (5) entlang der Gleitschiene (2) derart bilden, dass wenigstens ein zweites längliches, graduiertes Messelement (6) in einer Ebene angeordnet ist, die lotrecht zu der genannten ersten Längsrichtung des genannten ersten graduierten Messelements (2) ist, wobei die genannten Gleitschlitten (5) angeordnet sind, um zwischen der genannten Vielzahl von distalen Gurten (3) und der genannten Vielzahl von proximalen Gurten (4) positioniert zu sein.

## Claims

1. Device for the iterative measurement (1) of the circumference of an object, in particular a limb, comprising:
- a first longitudinal graduated measuring element (2) having a first longitudinal direction, arranged to be placed alongside the said object, in particular alongside the said limb and defining a slide (2),
- a distal strap (3) and a proximal strap (4) each located in a plane substantially perpendicular to the said longitudinal direction, and
- a slider (5) engaging with the said slide (2), linked to a second longitudinal graduated measuring element (6), the said slider (5) having an orifice for the slide to pass through (5') and being positioned between the said distal strap (3) and the said proximal strap (4),
wherein:
- the said slide (2) and a flexible strap having a section affixed alongside its length,
- the said passage orifice (5') is defined by four walls, at least two of which are parallel to one another and each of them forms a guide wall for the slider (5) along the slide (2) so as to fit the said second longitudinal graduated measuring element (6) in a plane perpendicular to the said first longitudinal direction,
- the said proximal strap (4) is linked to the slide (2) or a slider (5) engaging with the said slide other than that to which the said second graduated measuring element (6) is linked, the said proximal strap (4) being detachable or not,
- the said distal strap (3) is linked to the slide (2) or a slider (5) engaging with the said slide other than that to which the said second graduated measuring element (6) is linked, the said distal strap (3) being detachable or not, and where
- the said distal strap (3) and proximal strap (4) are the graduated longitudinal bands or those having a first (7) and a second (8) ends, each equipped with a closing (9) and having an open and a closed position, the said open position being a position where the first (7) and the second (8) ends are spaced from one another, whereas the closed position is a position in which the said first end (7) of the said longitudinal band goes over the said second end (8).

2. Measuring device (1) according to claim 1, where the said slide (2) and/or the said second graduated measuring element (6) can be wound.

3. Measuring device according to claim 1, where the said slide (2) and/or the said second graduated measuring element (6) is foldable, e.g.: in an S shape.

4. Measuring device (1) according to which any of the claims from 1 to 3, where there are multiple sliders (5).

5. Measuring device (1) according to which any of the claims from 1 to 4, where the said passage orifice (5') of the said slider (5) having a section similar to the slide section (2).

6. Measuring device (1) according to which any of the claims from 1 to 5, where the said slide (2) is a slide with a flexible material, such as flexible plastic or paper having sufficient tensile strength.

7. Measuring device (1) according to which any of the claims from 1 to 6, where the said second measuring element (6) linked to a slider (5) is detachable.

8. Measuring device(1) according to which any of the claims from 1 to 7, where the said closing means (9) is chosen from the group made up of a press button, a pushbutton or self-gripping means, e.g.: Velcro®.

9. Measuring device(1) according to which any of the claims from 1 to 8, where the said second measuring element (6) is provided, at its end not connected to the first measuring element (2), with a removable or non-removable end-piece (12) comprising a first pair of protrusions (13, 13') extending upwards in a direction perpendicular to the plane formed by the said second graduated measuring element (6) and/or a second pair of protrusions (14, 14') extending outward substantially in the same plane as that formed by the said second graduated measuring element (6).

10. Kit to create a measuring device according to any of the claims from 1 to 9, to measure the circumference of an object, in particular a limb, the said kit comprises:
- at least one first graduated measuring element (2) having a first longitudinal direction and defining a flexible slide (2) and being arranged to be affixed along a bodily segment,
- multiple second graduated measuring elements (6),
- multiple distal straps (3) arranged, each to be located in a plane substantially perpendicular to the said longitudinal direction and arranged to be linked to the slide (2) or a slider (5) other than that to which the said second graduated measurement element (6), the distal straps (3) being detachable or not, the said distal straps (3) being graduated longitudinal bands or those having a first (7) and a second (8) end, each equipped with a closing (9) and having an open and closed position, the said open position being a position where the first (7) and the second (8) ends are spaced from one another, whereas the closed position is a position in which the said first end (7) of the said longitudinal band goes over the said second end (8),
- multiple proximal straps (4) arranged, each to be located in a plane substantially perpendicular to the said longitudinal direction and placed to be linked to the slide (2) or a slider (5) other than that to which the said graduated second measurement element (6), the said proximal straps (4) being detachable or, the said proximal straps (4) being graduated longitudinal bands or having a first (7) and a second (8) end, each equipped with a closing (9) and having an open and closed position, the said open position being a position where the first (7) and the second (8) ends are spaced from one another, whereas the closed position is a position in which the said first end (7) of the said longitudinal band goes over the said second end (8), and
- multiple sliders (5) arranged to engage with the said slide (2) and having an orifice for the slide to pass through (5') defined by four walls, at least two of which are parallel to one another and each forms a guide wall for the slider (5) alongside the slide (2) so as to fit at least a second longitudinal graduated measuring element (6) in a plane perpendicular to the said first longitudinal direction of the said first graduated measuring element (2), the sliders (5) being arranged to be positioned between the said multiple distal straps (3) and the said multiple proximal straps (4).
